# EUROPEAN PATENT SPECIFICATION

(11) **EP 3 347 280 B1**
(45) Date of publication and mention of the grant of the patent: **14.08.2019**
(21) Application number: 16767191.6
(22) Date of filing: 09.09.2016
(51) Int. Cl.: B65B 61/20, B65C 9/12, B65C 9/14, B65C 9/16

(54) **APPARATUS FOR APPLYING LEAFLETS TO CONTAINERS**
VORRICHTUNG ZUM ANBRINGEN VON PROSPEKTEN AUF BEHÄLTERN
APPAREIL POUR APPLIQUER DES FEUILLETS À DES RÉCIPIENTS

(30) Priority: 11.09.2015 IT UB20153359
(43) Date of publication of application: 18.07.2018
(73) Proprietor: P.E. Labellers S.p.A., 46047 Porto Mantovano (MN) (IT)
(72) Inventor: SCHINELLI, Nicola, 46100 Mantova (IT)
(74) Representative: Modiano, Micaela Nadia
(86) International application number: PCT/EP2016/071273
(87) International publication number: WO 2017/042316

(56) References cited:
- WO-A1-2005/120961
- GB-A- 2 449 621
- US-A- 2 545 292
- US-A- 3 537 934
- US-A1- 2012 227 906
- US-B1- 6 167 935

## Description

The present invention relates to an apparatus for applying leaflets to containers.

It is known to apply to the external surface of containers of drugs or other products informational leaflets, commonly known as "outserts", which are normally constituted by a sheet that is folded in multiple folds and bears, printed thereon, information related to the product that is present in the corresponding container.

Currently, these leaflets are applied automatically by means of apparatuses (s. e.g. document GB 2 449 621 A) which generally have an applicator which moves intermittently in order to transfer in each instance a leaflet picked up from a corresponding magazine onto a corresponding container that arrives from an adapted conveyor.

The intermittent movement of the applicator makes these known apparatuses very slow and therefore unsuitable to be used along high-speed packaging lines.

The aim of the present invention is to provide an apparatus for applying leaflets to containers that can operate at the speeds of the most modern packaging lines.

Within this aim, an object of the present invention is to provide an apparatus for applying leaflets to containers that ensures high reliability in the operation for fixing the leaflets to the containers.

Another object of the invention is to provide an apparatus for applying leaflets to containers that has a simple structure and is competitive from an economic standpoint.

This aim, as well as these and other objects that will become better apparent hereinafter, are achieved by an apparatus for applying leaflets to containers, according to the invention, as defined in claim 1.

Further characteristics and advantages of the present invention will become better apparent from the following detailed description of a preferred but not exclusive embodiment of the apparatus according to the invention, illustrated by way of non-limiting example in the accompanying drawings, wherein:

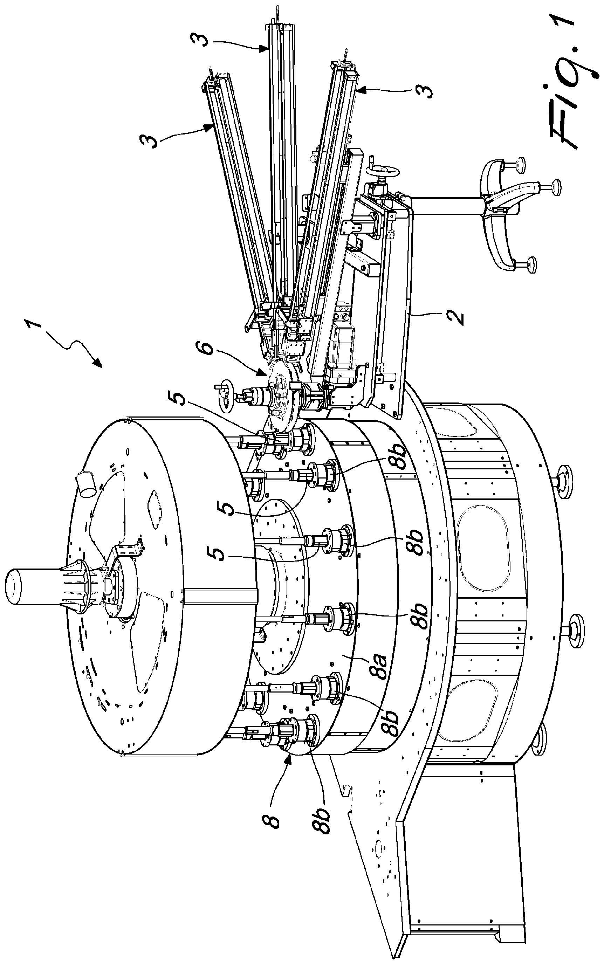

Figure 1 is a perspective view of an apparatus according to the invention;

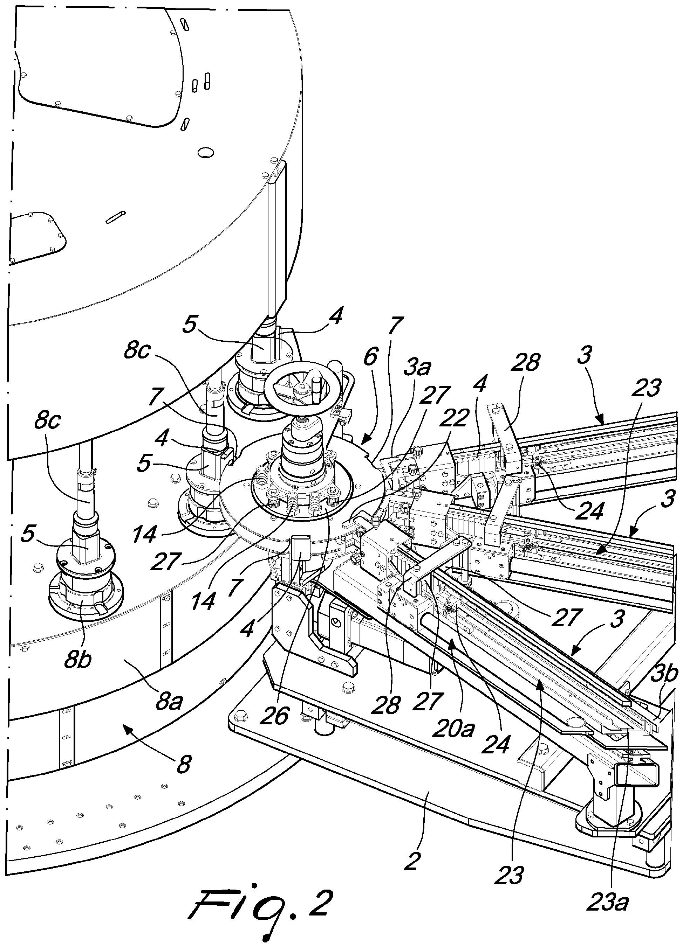

Figure 2 is a perspective view of a detail of the apparatus according to the invention;

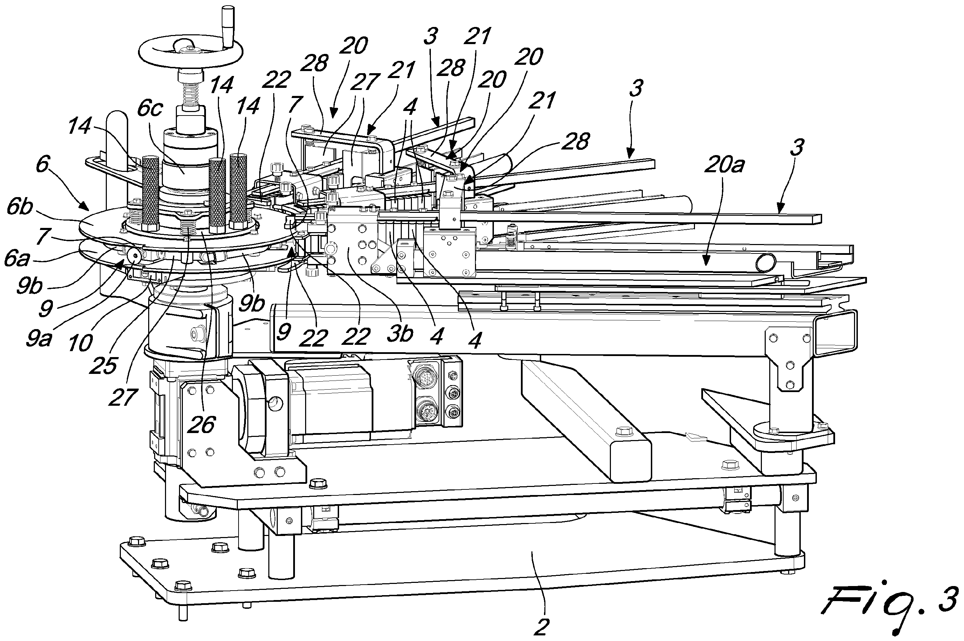

Figure 3 is a perspective view of a portion of the apparatus according to the invention;

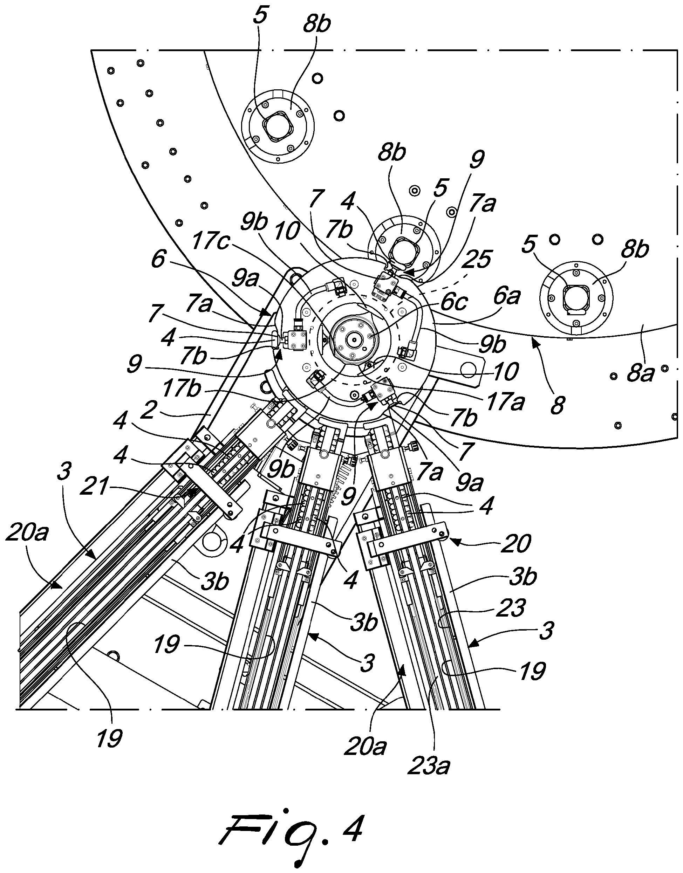

Figure 4 is a top plan view of the apparatus according to the invention, with parts omitted for the sake of simplicity and magazines shown in the inactive position;

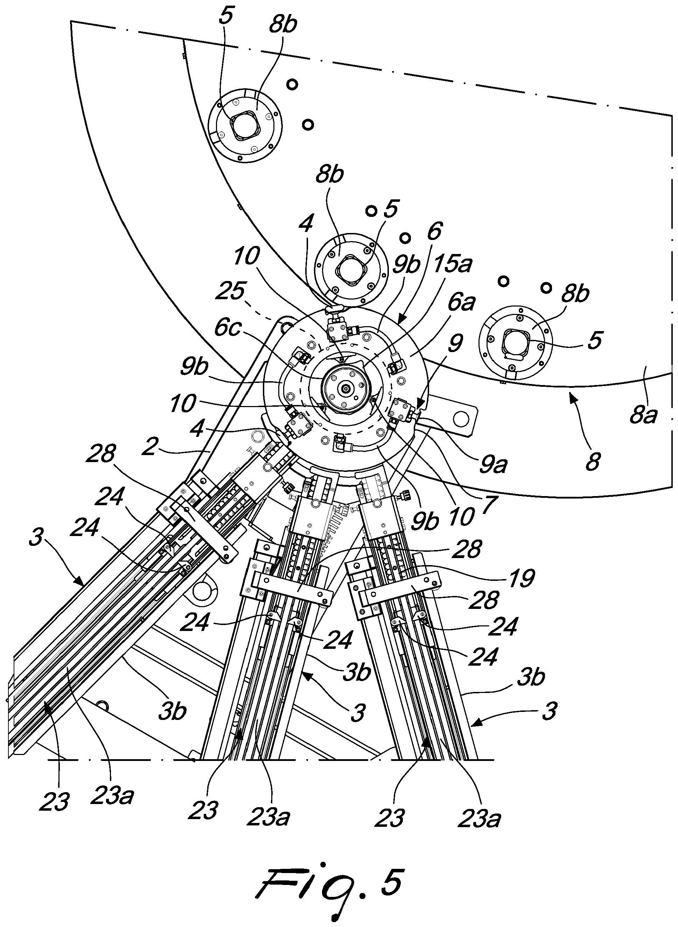

Figure 5 is a top plan view of the apparatus according to the invention with parts omitted for the sake of simplicity and with a magazine in the active position in order to allow an application drum to pick up leaflets from it;

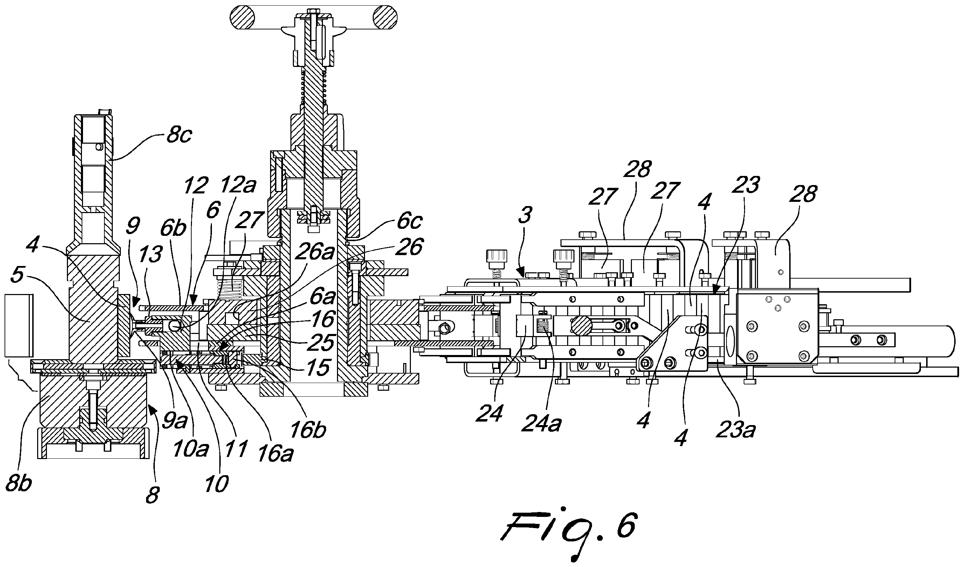

Figure 6 is a sectional view of the apparatus according to the invention;

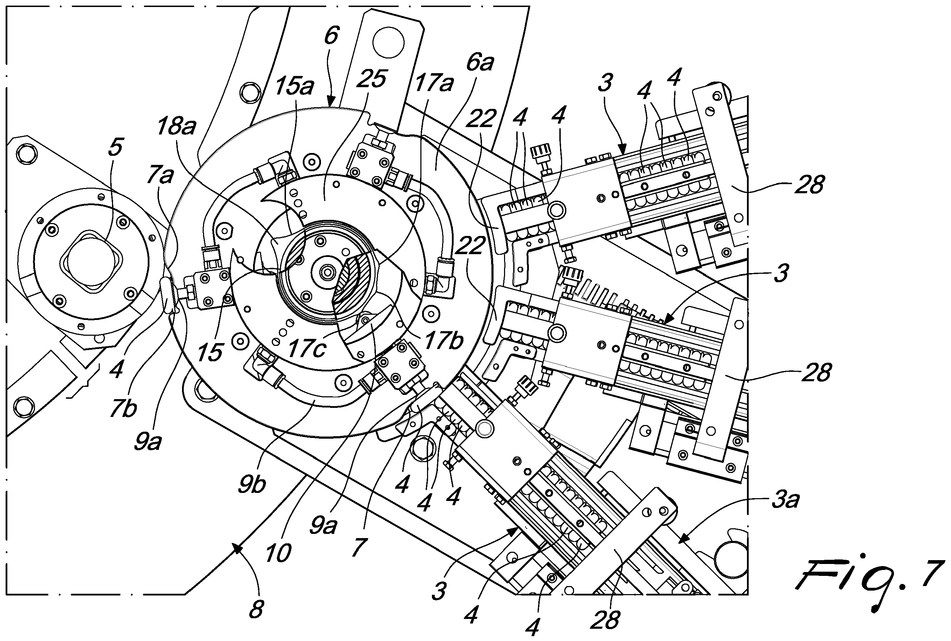

Figure 7 is an enlarged-scale top plan view of a portion of the apparatus according to the invention with parts omitted and others shown in cutout view.

With reference to the figures, an apparatus for applying leaflets to containers, designated generally by the reference numeral 1, comprises a fixed framework 2 which supports at least one magazine 3, which is intended to contain a plurality of leaflets 4 to be applied to containers 5 and is provided with at least one outlet 3a which is arranged so as to face the peripheral region of an application drum 6, which can rotate about its own axis with respect to the fixed framework 2.

In particular, the application drum 6 has, on its peripheral region, at least one accommodation seat 7 that is intended to receive at least one respective leaflet 4 picked up at the outlet 3a of the magazine 3 and meant to be transferred onto at least one respective container 5 moved by a conveyor 8, which is arranged laterally adjacent to the application drum 6 and is constituted for example by a carousel 8a provided conveniently with pans 8b on which individual containers 5 are rested and, optionally, with locking elements 8c for the containers 5, each arranged above a respective pan 8b in order to clamp a corresponding container 5.

According to the invention, the application drum 6 can be actuated with a continuous rotary motion about its own axis and is provided, at the accommodation seat 7, with means 9 for retaining the leaflets 4 picked up from the magazine 3.

Also according to the invention, the means 9 for retaining the leaflets 4 can be moved on command between a condition that lies close to the axis of the application drum 6, in order to allow the installation of the leaflets 4 in the accommodation seat 7, and at least one condition that is spaced from the axis of the application drum 6 in order to pick up the leaflets from the outlet 3a of the magazine 3 and transfer the leaflets 4 onto the respective container 5.

With reference to the embodiment shown in the figures, it is possible advantageously to provide at least two distinct magazines 3, which are arranged so as to be mutually angularly spaced around the application drum 6.

For example, as shown, there can be three distinct magazines 3, but their number can also be different according to the requirements.

Conveniently, the magazines 3 can be arranged selectively between an active position, in which they are closer, with the corresponding outlet 3a, to the peripheral region of the application drum 6, in order to allow the latter to pick up the leaflets 4 contained therein, and an inactive position, in which they are spaced, with the respective outlet 3a, from the peripheral region of the application drum 6.

Again with reference to the illustrated embodiment, at the peripheral region of the application drum 6 there is conveniently a plurality of accommodation seats 7, which are distributed so as to be mutually spaced around the axis of the application drum 6 and are intended to each receive a respective leaflet 4, which is picked up at the outlet 3a of the magazines 3. In this case, respective retention means 9 are provided at each one of the accommodation seats 7.

In greater detail, each accommodation seat 7 conveniently has, at a front end, along the direction of rotation of the application drum 6, a guiding portion 7a that is inclined progressively toward the axis of the application drum 6 as one proceeds toward the rear end of the accommodation seat 7, so as to facilitate the entry of the leaflets 4 in the accommodation seats 7.

Advantageously, at its rear end, each accommodation seat 7 has an abutment portion 7b that is extended substantially at right angles to the axis of the application drum 6 in order to allow traction of the leaflets 4 by the application drum 6 in its rotation about its own axis.

Conveniently, the application drum 6 has a base body that is mounted so that it can rotate with respect to the fixed framework 2.

Advantageously, the retention means 9 of each accommodation seat 7 are supported by a respective carriage 10, which is located at the corresponding accommodation seat 7 and is mounted on the base body of the application drum 6 so that it can move along a direction that is substantially perpendicular to the axis of said application drum.

Advantageously, the retention means 9 can be constituted by at least one respective suction device, constituted conveniently by at least one respective sucker 9a which is connected to respective vacuum creation means, which are constituted for example by a vacuum pump or other similar device, not shown, by means of a corresponding suction duct 9b.

In greater detail, the base body of the application drum 6 can, for example, be constituted by a pair of plates 6a and 6b which face each other, are substantially annular and are arranged around a central body 6c, which is coaxial to the axis of the application drum 6. Conveniently, each carriage 10 is mounted so that it can slide on linear guides 10a which extend radially with respect to the axis of the application drum 6 and are formed on one of the plates 6a, 6b, for example the one designated by the reference numeral 6a in the drawings.

In particular, the carriages 10 and the corresponding linear guides 10a are arranged on the face of the plate 6a that is directed in the opposite direction with respect to the other plate 6b.

Conveniently, each carriage 10 is connected, by means of a respective slot 11 formed in the plate 6a, to a corresponding block 12, which is arranged between the plates 6a and 6b and on which the corresponding sucker 9a is mounted.

In particular, each block 12 forms internally a suction chamber 12a, which is connected to the corresponding sucker 9a, for example by means of a connecting nipple 13, and to the respective vacuum creation means, by means of the suction ducts 9b, which are arranged conveniently between the plates 6a and 6b and are connected to corresponding vacuum ports 14 arranged on the central body 6c.

Preferably, the suction ducts 9b are connected to a first vacuum distribution body 25, which is integral with the application drum 6 and is coupled to a second vacuum distribution body 26, which is integral with the central body 6c. In particular, the first vacuum distribution body 25 has, at each suction duct 9b, a respective passage opening intended to be connected to a corresponding vacuum distribution groove 26a, which is formed in the second vacuum distribution body 26, which is connected to a corresponding vacuum port 14 and is extended along a circular arc which is extended conveniently at least for the portion of path of the application drum 6 in which the intervention of the corresponding sucker 9a is required.

Advantageously, the second vacuum distribution body 26 is made of plastic material and is conveniently kept in hermetic contact against the first vacuum distribution body 25 by way of pusher springs 27.

Advantageously, the movement of the carriages 10 along the respective linear guides 10a is provided by providing at least one contoured body 15, which is integral with the fixed framework 2 and in particular is fixed for example to the central body 6c and is provided with a cam-like profile 15a which is extended around the axis of the application drum 6 and is engaged slidingly by the carriages 10 in their movement integrally with the application drum 6.

In particular, the sliding of the carriages 10 on the cam-like profile 15a of the contoured body 15 allows to produce the movement of the retention means 9 and more particularly of the suckers 9a supported by the carriages 10 between said closer condition and at least one condition which is spaced from the axis of the application drum 6 in contrast with, or by virtue of the action of, elastic return means 16.

For example, the elastic return means 16 comprise, for each carriage 10, at least one respective spring 16a, which acts along a direction that is substantially radial with respect to the axis of the application drum 6, between the corresponding carriage 10 and a corresponding abutment 16b that is integral with the base body of the application drum 6 and in particular, according to the illustrated example, with the plate 6a.

Conveniently, the cam-like profile 15a has variations in the distance from the axis of the application drum 6 substantially at the angular position in which the magazines 3 are located with respect to the axis of the application drum and the conveyor 8, or, more conveniently, in a position that is slightly upstream with respect to the angular position of the magazines 3 along the direction of rotation of the application drum 6.

According to the illustrated embodiment, these distance variations comprise first protrusions 17a, 17b, 17c, which are arranged at the magazines 3 and protrude, with respect to the remaining part of the contoured body 15, in the direction of the peripheral region of the application drum 6, and at least one second protrusion 18a, which is arranged at the conveyor 8 and protrudes more than the first protrusions 17a, 17b and 17c, so that the engagement of the carriages 10 with the second protrusion 18a can cause a greater spacing of the retention means 9 from the axis of the application drum 6 with respect to the situation in which the carriages engage the first protrusions 17a, 17b, 17c.

Conveniently, each one of the magazines 3 has at least one respective containment chamber 19 for a plurality of leaflets 4 to be applied and feeder means which allow to arrange one leaflet 4 at a time at the respective outlet 3a.

In greater detail, the containment chamber 19 of each magazine 3 is extended conveniently along a direction that is substantially radial with respect to the axis of the application drum 6 and it is possible to insert therein the leaflets 4 to be applied, one next to the other.

In particular, the outlet 3a of the magazines 3 is arranged at the end that is directed toward the application drum 6 of the corresponding containment chamber 19.

Each one of the magazines 3 can translate selectively with respect to the fixed framework 2 along a direction that is substantially perpendicular to the axis of the application drum, so as to be able to pass from the inactive position to the active position and vice versa.

More particularly, each magazine 3 conveniently has a frame 3b with an elongated extension that forms internally the containment chamber 19 and can slide axially, with respect to the fixed framework 2, between said inactive position and said active position of the magazine 3.

The feeder means of each magazine 3 are constituted by respective pushers 20, preferably constituted by linear actuation means 20a, conveniently provided by at least one respective piston with magnetic rings, which act on pusher elements 21 intended to rest against the row of leaflets 4 inserted in the corresponding containment chambers 19 so as to push them toward the corresponding outlet 3a.

Conveniently, the magazines 3 have, at the corresponding outlets 3a, means for blocking the leaflets 4, which are constituted for example by a pair of abutment retainers 22, which are mutually opposite and face the corresponding outlet 3a and against which the first leaflet 4 that is present at the outlet 3a of the magazines 3 rests when the magazines are in the inactive position.

The abutment retainers 22 are conveniently mutually spaced along a direction that is substantially parallel to the axis of the application drum 6. In particular, the distance between the abutment retainers 22 is advantageously greater than the dimension in the axial direction of the application drum 6.

With the magazines 3 in the active position, the abutment retainers 22 are arranged beyond the edge of the application drum 6, toward the axis thereof, and the leaflet 4 that in each instance is located at the outlet 3a makes contact with the peripheral region of the application drum 6 and more particularly with the perimetric edge of the plates 6a and 6b, waiting to be picked up by one of the accommodation seats 7 of the application drum 6 and by the corresponding retention means 9. In practice, with the magazines 3 in the active position, the leaflet 4 that is at the outlet 3a of the corresponding magazine is no longer blocked, in its advancement under the thrust of the pushers 20, by contact against the abutment retainers 22 but by contact against the edge of the application drum 6, ready to be pulled by the application drum 6 upon the arrival of one of the accommodation seats 7.

Advantageously, inside the containment chamber 19 formed by the frame 3b of each magazine 3 it is possible to insert axially a holder 23 of new leaflets 4, which allows rapid filling of the magazines 3 when the leaflets 4 contained therein are being depleted or are completely depleted.

Each holder 23 comprises a slider 23a which supports a row of new leaflets 4 to be loaded into the corresponding magazine 3 and can be made to slide within the containment chamber 19 of the magazine 3 to be reloaded, inserting it from the end of the containment chamber 19 that is opposite to the one in which the outlet 3a of the magazine is formed, until the leaflets 4 of the holder 23 are brought against any leaflets 4 remaining within the magazine 3.

Conveniently, at the end of each magazine 3 intended to be directed toward the outlet 3a of the magazine 3 there is an exit opening for the leaflets 4 carried by the magazine 3, in which there are means for blocking the leaflets 4 in the magazine 3 which are capable of deactivating automatically when the leaflets 4 of the magazine 3 are pushed by the pusher 20 of the magazine 3 toward the outlet 3a thereof.

The blocking means are constituted for example by a pair of blocking levers 24, which are supported by the slider 23a of the holder 23 and are arranged on mutually opposite sides with respect to the exit opening of the holder 23 and can oscillate in contrast with elastic return springs 24a between a closed condition, in which they prevent the exit of the leaflets 4 from the corresponding holder 23, and an open condition, which is assumed under the thrust received by the leaflets 4 of the holder 23, in the direction of the outlet 3a of the magazine, as a consequence of the actuation of the pusher 20 of the magazine 3, in which they allow the crossing of the exit opening of the holder 23 by the leaflets 4 inserted inside it.

Conveniently, the pusher elements 21 of the pushers 20 of the magazines 3 are constituted by a pair of rotatable flaps 27, which are mutually opposite and are supported by a sliding element 28, which can be actuated for translation by the linear actuation means 20a along the axis of the containment chamber 19 of the corresponding magazine 3 both in the direction of the outlet 3a of the magazine 3 and in the opposite direction.

In particular, the rotatable flaps 27 can pass, in contrast with elastic return means, from an engagement position, in which they are capable of engaging the rear end of the row of leaflets 4 that is directed in the opposite direction with respect to the outlet 3a of the magazine 3, to a disengaged condition, which they assume automatically when the corresponding sliding element 28 is made to retract by the linear actuation means 20a in the opposite direction with respect to the outlet 3a of the magazine 3, after a new holder 23 of leaflets 4 has been inserted behind the sliding element 28 so as to allow the sliding element 28 to reach and move beyond the rear end of the row of leaflets 4 that are present in the inserted holder 23.

Conveniently, glue application means, not shown, are also provided which allow to deposit on the leaflets 4 picked up by the application drum or directly on the containers 5 a layer of glue which allows the adhesion of the leaflets 4 to the containers 5.

Operation of the apparatus according to the invention is as follows.

One of the magazines 3, loaded with the leaflets 4 to be applied, is arranged in the active position and the application drum 6 is actuated with a continuous rotary motion about its own axis.

The leaflet 4 that is located at the outlet 3a of the magazine 3 arranged in the active position, free from the engagement of the abutment retainers 22, is pushed by the pusher 20 of the corresponding magazine 3 against the peripheral region of the application drum 6, waiting for the arrival of an accommodation seat 7.

As soon as an accommodation seat 7 reaches the position in which the magazine 3 is located in the active position, the leaflet 4 that is located at the outlet 3a of the magazine 3 begins to engage the guiding portion 7a of the incoming accommodation seat 7, so as to progressively enter it.

In the meantime, the carriages 10, pulled by the base body of the application drum 6 in its rotary motion about its own axis, slide on the cam-like profile 15a of the contoured body 15 and undergo, at the first protrusions 17a, 17b, 17c, a translation in a radial direction, in contrast with the action of the corresponding spring 16a, so as to move the corresponding sucker 9a from a closer condition to a spaced condition with respect to the axis of the application drum 6.

In this manner, the suckers 9a of the carriages 10, also by way of the action of the vacuum creation means connected thereto, can make contact with and adhere perfectly to the leaflet 4 that is located at the outlet 3a of the magazine 3 that is arranged in the active position and which, as a consequence of its sliding along the guiding portion 7a, has progressively entered the accommodation seat 7 that has reached its position.

As soon as the carriages 10 abandon the first protrusion that is located at the magazine 3 arranged in the active position, they are returned by the action of the spring 16a to a condition that lies closer to the axis of the application drum.

In this manner, the installation in the accommodation seat 7 of the picked up leaflet 4 is also completed, and said leaflet is thus retained by the corresponding sucker 9a adjacent to the bottom of the accommodation seat 7 and is therefore pulled in the rotation of the application drum 6 by its resting contact against the abutment portion 7b.

Continuing in the rotation of the application drum 6, the accommodation seat 7 with the picked up leaflet 4 inside it reaches the position of the conveyor 8 of the containers 5.

At this point, the corresponding carriage 10 engages the second protrusion 18a of the contoured body 15, thus undergoing a displacement from the closer condition to a spaced condition with respect to the axis of the application drum 6.

In this manner, the leaflet 4 arranged in the accommodation seat 7 is moved into contact with and pressed against a container 5 that is arriving on the conveyor 8 and subsequently the vacuum creation means connected to the corresponding sucker 9a are deactivated, so that the sucker 9a can detach from the leaflet 4.

In particular, the deactivation of the vacuum creation means is achieved because the passage opening of the first vacuum distribution body 25 connected to the sucker 9a has moved beyond the corresponding vacuum distribution groove 26a formed in the second vacuum distribution body 26.

Thanks to a layer of glue applied previously to the leaflet 4 or to the container 5 by adapted application means, the leaflet 4 can therefore adhere perfectly to the container 5 and be transferred onto it.

The continuation of the rotation of the application drum 6 about its own axis causes the disengagement of the carriage 10 from the second protrusion 18a and its return to the condition that lies closer to the axis of the application drum 6 by way of the action of the corresponding spring 16a.

At this point, the vacuum creation means connected to the sucker 9a supported by the carriage 10 that has returned to the condition that lies closer to the application drum 6 are reactivated so as to prepare themselves to pick up a new leaflet 4. This reactivation is achieved by virtue of the new reaching of the corresponding vacuum distribution groove 26a that is formed in the second vacuum distribution body 26 by the passage opening provided in the first vacuum distribution body 25 and connected to the sucker 9a, during the rotation of the application drum 6 about its own axis.

When all the leaflets 4 of the magazine 3 in the active position have been picked up, another magazine 3, filled with leaflets 4, is moved to the active position and the empty one is returned to the inactive position so that it can be reloaded with new leaflets, without loss of production, by using a full holder 23.

In practice it has been found that the invention is capable of achieving fully the intended aim and objects.

All the characteristics of the invention indicated above as advantageous, convenient or the like may also be omitted or be replaced with equivalents.

The individual characteristics described with reference to general teachings or particular embodiments may all be present in other embodiments or may replace characteristics in these embodiments.

The invention thus conceived is susceptible of numerous modifications and variations, all of which are within the scope of the appended claims.

In practice, the materials used, so long as they are compatible with the specific use, as well as the dimensions and shapes, may be any according to requirements.

All the details may further be replaced with other technically equivalent elements.

The disclosures in Italian Patent Application No. 102015000050627 (UB2015A003559) from which this application claims priority are acknowledged.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An apparatus for applying leaflets (4) to containers (5), comprising a fixed framework (2) supporting at least one magazine (3) adapted to contain a plurality of leaflets (4) and having at least one outlet (3a) for said leaflets that is arranged so as to face the peripheral region of an application drum (6), which can rotate about its own axis, with respect to said fixed framework (2), and having, on its own peripheral region, at least one accommodation seat (7) for at least one respective leaflet (4) picked up from said outlet (3a) and intended to be transferred onto at least one respective container (5) moved by a conveyor (8), **characterized in that** said application drum (6) can be actuated with a continuous rotary motion about its own axis and is provided, at said at least one accommodation seat (7), with means (9) for retention of said at least one leaflet (4) that can be moved on command between a condition that lies close to the axis of said application drum (6), in order to allow the installation of said at least one leaflet (4) in said at least one accommodation seat (7), and a condition that is spaced from the axis of said application drum (6), in order to pick up said at least one leaflet (4) from said outlet (3a) and to transfer said at least one leaflet (4) onto the respective container (5).

2. The apparatus according to claim 1, **characterized in that** said at least one accommodation seat (7) has, at a front end thereof according to the rotation direction of said application drum (6), a guiding portion (7a) that is inclined progressively toward the axis of said application drum (6), in order to allow the entry of said at least one leaflet (4) in said at least one accommodation seat (7), and, at its rear end, an abutment portion (7b) that is extended substantially at right angles to the axis of said application drum (6) in order to allow traction of said at least one leaflet (4) by said application drum (6) in its rotation about its own axis.

3. The apparatus according to one or more of the preceding claims, **characterized in that** said application drum (6) has a base body that can rotate about its own axis with respect to said fixed framework (2), said retention means (9) being supported by a respective carriage (10), arranged at said at least one accommodation seat (7) and mounted so that it can move on said base body of said application drum (6) along a direction that is substantially perpendicular to the axis of said application drum (6).

4. The apparatus according to one or more of the preceding claims, **characterized in that** said retention means (9) comprise at least one suction device.

5. The apparatus according to claim 4, **characterized in that** said at least one suction device comprises at least one sucker (9a) connected to means for creating vacuum.

6. The apparatus according to one or more of the preceding claims, **characterized in that** it comprises at least one contoured body (15) with a cam-like profile (15a) that is extended around the axis of said application drum (6) and is engaged slidingly by said carriage (10) in its movement integrally with said application drum (6), in order to cause the movement of said retention means (9) between said closer condition and said spaced condition in contrast with, or by the action of, elastic return means (16).

7. The apparatus according to one or more of the preceding claims, **characterized in that** it comprises, at the peripheral region of said application drum (6), a plurality of accommodation seats (7) distributed around the axis of said application drum (6) and intended to receive each a respective leaflet (4) picked up at said at least one outlet (3a) of said at least one magazine (3), respective retention means (9) being provided at each one of said accommodation seats (7).

8. The apparatus according to one or more of the preceding claims, **characterized in that** it comprises at least two magazines (3) that are mutually arranged so as to be angularly spaced around said application drum (6).

9. The apparatus according to claim 8, **characterized in that** said magazines (3) can be arranged selectively between an active position, in which they are closer, with their outlet (3a), to the peripheral region of said application drum (6), and an inactive position, in which they are spaced, with the respective exit (3a), from the peripheral region of said application drum (6).

10. The apparatus according to claims 8 or 9, **characterized in that** each one of said magazines (3) has at least one respective chamber (19) for containing a plurality of leaflets (4) to be applied and means for feeding a leaflet (4) at a time at the respective outlet (3a) of said magazines (3).

11. The apparatus according to claim 6, **characterized in that** said cam-like profile (15a) has variations in its distance from the axis of said application drum (6) that are substantially at the angular position of said magazines (3) and of said conveyor (8) with respect to the axis of said application drum (6).

## Patentansprüche

1. Vorrichtung zum Anbringen von Prospekten (4) auf Behälter (5) mit einem festen Rahmen (2), der mindestens ein Magazin (3) trägt, das mehrere Prospekte (4) aufnehmen kann und mindestens einen Auslass (3a) für die Prospekte (4) aufweist, welcher so angeordnet ist, dass er dem Umfangsbereich einer Auftragstrommel (6) zugewandt ist, die sich in Bezug auf den festen Rahmen (2) um ihre eigene Achse drehen kann und auf ihrem eigenen Umfangsbereich mindestens einen Aufnahmesitz (7) für mindestens ein jeweiliges Prospekt (4) aufweist, das von dem Auslass (3a) aufgenommen wurde und dazu bestimmt ist, auf mindestens einen jeweiligen Behälter (5) übertragen zu werden, der von einem Förderer (8) bewegt wird, **dadurch gekennzeichnet, dass** die Auftragstrommel (6) mit einer kontinuierlichen Drehbewegung um ihre eigene Achse angetrieben werden kann und an dem mindestens einen Aufnahmesitz (7) Mittel (9) zum Zurückhalten des mindestens einen Prospekts (4) aufweist, welche Mittel auf Befehl zwischen einem Zustand, der in der Nähe der Achse der Auftragstrommel (6) liegt, um die Anordnung des mindestens einen Prospekts (4) in dem mindestens einen Aufnahmesitz (7) zu ermöglichen, und einem Zustand, der von der Achse der Auftragstrommel (6) beabstandet ist, um das mindestens eine Prospekt (4) aus dem Auslass (3a) aufzunehmen und das mindestens eine Prospekt (4) auf den jeweiligen Behälter (5) zu übertragen, bewegt werden können.

2. Vorrichtung nach Anspruch 1, **dadurch gekennzeichnet, dass** der mindestens eine Aufnahmesitz (7) an einem gemäß der Drehrichtung der Auftragstrommel (6) vorderen Ende hiervon einen Führungsabschnitt (7a), der progressiv in Richtung der Achse der Auftragstrommel (6) geneigt ist, um den Eintritt des mindestens einen Prospekts (4) in den mindestens einen Aufnahmesitz (7) zu ermöglichen, und an seinem hinteren Ende einen Anschlagsabschnitt (7b) aufweist, der im Wesentlichen rechtwinklig zur Achse der Auftragstrommel (6) verlängert ist, um den Transport des mindestens einen Prospekts (4) durch die Auftragstrommel (6) in ihrer Drehung um ihre eigene Achse zu ermöglichen.

3. Vorrichtung nach einem oder mehreren der vorhergehenden Ansprüche, **dadurch gekennzeichnet, dass** die Auftragstrommel (6) einen Grundkörper aufweist, der in Bezug auf den festen Rahmen (2) um seine eigene Achse drehbar ist, wobei die Rückhaltemittel (9) abgestützt sind durch einen jeweiligen Schlitten (10), der an dem mindestens einen Aufnahmesitz (7) angeordnet und so angebracht ist, dass er sich auf dem Grundkörper der Auftragstrommel (6) entlang einer Richtung bewegen kann, die im Wesentlichen senkrecht zu der Achse der Auftragstrommel (6) ist.

4. Vorrichtung nach einem oder mehreren der vorhergehenden Ansprüche, **dadurch gekennzeichnet, dass** die Rückhaltemittel (9) mindestens eine Saugvorrichtung umfassen.

5. Vorrichtung nach Anspruch 4, **dadurch gekennzeichnet, dass** die mindestens eine Saugvorrichtung mindestens einen Sauger (9a) umfasst, der mit Mitteln zum Erzeugen von Vakuum verbunden ist.

6. Vorrichtung nach einem oder mehreren der vorhergehenden Ansprüche, **dadurch gekennzeichnet, dass** sie mindestens einen Konturkörper (15) mit einem nockenartigen Profil (15a) umfasst, der sich um die Achse der Auftragstrommel (6) erstreckt und durch den Schlitten (10) in seiner Bewegung integral mit der Auftragstrommel (6) gleitend in Eingriff steht, um die Bewegung der Rückhaltemittel (9) zwischen dem näheren Zustand und dem beabstandeten Zustand im Widerspiel mit oder durch die Wirkung von elastischen Rückholmitteln (16) zu bewirken.

7. Vorrichtung nach einem oder mehreren der vorhergehenden Ansprüche, **dadurch gekennzeichnet, dass** sie am Umfangsbereich der Auftragstrommel (6) mehrere Aufnahmesitze (7) aufweist, die um die Achse der Auftragstrommel (6) verteilt und vorgesehen sind, sodass jeder jeweils ein an dem mindestens einen Auslass (3a) des mindestens einen Magazins (3) aufgenommenes Prospekt (4) aufnimmt, wobei an jedem der Aufnahmesitze (7) entsprechende Rückhaltemittel (9) vorgesehen sind.

8. Vorrichtung nach einem oder mehreren der vorhergehenden Ansprüche, **dadurch gekennzeichnet, dass** sie mindestens zwei Magazine (3) umfasst, die so zueinander angeordnet sind, dass sie einen Winkelabstand um die Auftragstrommel (6) aufweisen.

9. Vorrichtung nach Anspruch 8, **dadurch gekennzeichnet, dass** die Magazine (3) ausgewählt zwischen einer aktiven Position, in der sie mit ihrem Auslass (3a) näher am Umfangsbereich der Auftragstrommel (6) liegen, und einer inaktiven Position, in der sie mit dem jeweiligen Auslass (3a) vom Umfangsbereich der Auftragstrommel (6) beabstandet sind, angeordnet werden können.

10. Vorrichtung nach Anspruch 8 oder 9, **dadurch gekennzeichnet, dass** jedes der Magazine (3) mindestens eine entsprechende Kammer (19) zum Aufnehmen mehrerer aufzutragender Prospekte (4) und Mittel zum Zuführen eines Prospekts (4) jeweils an dem jeweiligen Auslass (3a) der Magazine (3) aufweist.

11. Vorrichtung nach Anspruch 6, **dadurch gekennzeichnet, dass** das nockenartige Profil (15a) in seinem Abstand von der Achse der Auftragstrommel (6) Schwankungen aufweist, die im Wesentlichen in der Winkelposition der Magazine (3) und des Förderers (8) mit Bezug auf die Achse der Auftragstrommel (6) liegen.

## Revendications

1. Appareil pour appliquer des feuillets (4) sur des contenants (5), comportant un bâti fixe (2) supportant au moins un magasin (3) adapté pour contenir une pluralité de feuillets (4) et ayant au moins une sortie (3a) pour lesdits feuillets qui est agencée de manière à faire face à la zone périphérique d'un tambour d'application (6), qui peut tourner autour de son axe propre, par rapport audit bâti fixe (2), et ayant, au niveau de sa propre zone périphérique, au moins un siège de réception (7) pour au moins un feuillet (4) respectif pris à partir de ladite sortie (3a) et destiné à être transféré sur au moins un contenant (5) respectif déplacé par un convoyeur (8), **caractérisé en ce que** ledit tambour d'application (6) peut être actionné avec un mouvement rotatif continu autour de son axe propre et est pourvu, au niveau dudit au moins un siège de réception (7), de moyens (9) pour la retenue dudit au moins un feuillet (4) qui peut être déplacé sur commande entre un état qui est proche de l'axe dudit tambour d'application (6), afin de permettre l'installation dudit au moins un feuillet (4) dans ledit au moins un siège de réception (7), et un état qui est espacé de l'axe dudit tambour d'application (6), afin de prendre ledit au moins un feuillet (4) à partir de ladite sortie (3a) et de transférer ledit au moins un feuillet (4) sur le contenant (5) respectif.

2. Appareil selon la revendication 1, **caractérisé en ce que** ledit au moins un siège de réception (7) a, au niveau d'une extrémité avant de celui-ci dans le sens de rotation dudit tambour d'application (6), une partie de guidage (7a) qui est inclinée progressivement vers l'axe dudit tambour d'application (6), afin de permettre l'entrée dudit au moins un feuillet (4) dans ledit au moins un siège de réception (7), et, au niveau de son extrémité arrière, une partie de butée (7b) qui s'étend sensiblement à angles droits par rapport à l'axe dudit tambour d'application (6) afin de permettre une traction dudit au moins un feuillet (4) par ledit tambour d'application (6) dans sa rotation autour de son axe propre.

3. Appareil selon une ou plusieurs des revendications précédentes, **caractérisé en ce que** ledit tambour d'application (6) a un corps de base qui peut tourner autour de son axe propre par rapport audit bâti fixe (2), lesdits moyens de retenue (9) étant supportés par un chariot (10) respectif, agencé sur ledit au moins un siège de réception (7) et monté de sorte qu'il peut se déplacer sur ledit corps de base dudit tambour d'application (6) le long d'une direction qui est sensiblement perpendiculaire à l'axe dudit tambour d'application (6).

4. Appareil selon une ou plusieurs des revendications précédentes, **caractérisé en ce que** lesdits moyens de retenue (9) comportent au moins un dispositif d'aspiration.

5. Appareil selon la revendication 4, **caractérisé en ce que** ledit au moins un dispositif d'aspiration comporte au moins une ventouse (9a) reliée à des moyens destinés à créer un vide.

6. Appareil selon une ou plusieurs des revendications précédentes, **caractérisé en ce qu'**il comporte au moins un corps à contour façonné (15) avec un profil analogue à une came (15a) qui s'étend autour de l'axe dudit tambour d'application (6) et vient en contact de manière glissante avec ledit chariot (10) dans son mouvement solidaire dudit tambour d'application (6), afin d'entraîner le mouvement desdits moyens de retenue (9) entre ledit état rapproché et ledit état espacé en opposition à des moyens de retour élastique (16) ou par l'action de ceux-ci.

7. Appareil selon une ou plusieurs des revendications précédentes, **caractérisé en ce qu'**il comporte, au niveau de la zone périphérique dudit tambour d'application (6), une pluralité de sièges de réception (7) répartis autour de l'axe dudit tambour d'application (6) et destinés à recevoir chacun un feuillet (4) respectif pris au niveau de ladite au moins une sortie (3a) dudit au moins un magasin (3), des moyens de retenue (9) respectifs étant prévus sur chacun desdits sièges de réception (7).

8. Appareil selon une ou plusieurs des revendications précédentes, **caractérisé en ce qu'**il comporte au moins deux magasins (3) qui sont mutuellement agencés de manière à être angulairement espacés autour dudit tambour d'application (6).

9. Appareil selon la revendication 8, **caractérisé en ce que** lesdits magasins (3) peuvent être agencés sélectivement entre une position active, dans laquelle ils sont rapprochés, avec leur sortie (3a), de la zone périphérique dudit tambour d'application (6), et une position inactive, dans laquelle ils sont espacés, avec la sortie (3a) respective, de la zone périphérique dudit tambour d'application (6).

10. Appareil selon les revendications 8 ou 9, **caractérisé en ce que** chacun desdits magasins (3) a au moins une chambre (19) respective pour contenir une pluralité de feuillets (4) à appliquer et des moyens pour acheminer un feuillet (4) d'une traite au niveau de la sortie (3a) respective desdits magasins (3).

11. Appareil selon la revendication 6, **caractérisé en ce que** ledit profil analogue à une came (15a) a des variations de sa distance par rapport à l'axe dudit tambour d'application (6) qui sont sensiblement dans la position angulaire desdits magasins (3) et dudit convoyeur (8) par rapport à l'axe dudit tambour d'application (6).
